# EUROPEAN PATENT APPLICATION

(11) **EP 4 420 525 A1**
(43) Date of publication of application: **28.08.2024**
(21) Application number: 22883601.1
(22) Date of filing: 19.10.2022
(51) Int. Cl.: A23L 33/10, A61K 31/439, A61K 45/00, A61P 3/00, A61P 43/00

(54) **COMPOSITION USED TO PREVENT OR SUPPRESS REFEEDING SYNDROME**

(30) Priority: 19.10.2021 JP 2021171158
(71) Applicant: Otsuka Pharmaceutical Factory, Inc., Naruto-shi, Tokushima 772-8601 (JP)
(72) Inventor: YAMAOKA, Ippei, Naruto-shi, Tokushima 772-8601 (JP)
(74) Representative: Hoffmann Eitle
(86) International application number: PCT/JP2022/038927
(87) International publication number: WO 2023/068297

(57) **Abstract**

The present invention addresses the problem of providing a composition that is for use in preventing or suppressing refeeding syndrome. Provided as a means for solving the problem is a composition that is for use in preventing or suppressing refeeding syndrome, the composition containing an mTOR inhibitor.

## Description

### Technical Field

The present invention relates to a composition for use in preventing or suppressing refeeding syndrome.

### Background Art

"Refeeding syndrome" is a general term for a series of metabolic complications that develop as a result of providing aggressive nutritional support for subjects in a chronically malnourished state (Non-patent Literature (NPL) 1 and NPL 2). In other words, refeeding syndrome develops due to sudden start of nutritional therapy in subjects in an undernourished state. Subjects in an undernourished state transported to healthcare facilities in an emergency are particularly vulnerable to developing refeeding syndrome and often have difficulty in nutritional support.

Specific examples of symptoms of refeeding syndrome include hypophosphatemia, hypokalemia, hypomagnesemia, and vitamin B1 deficiency.

### Citation List

### Non-patent Literature

NPL 1: Hisham Mehanna et al., "Refeeding syndrome - awareness, prevention and management," 2009, Head & Neck Oncology, 1:4
NPL 2: Stanga Z et al., "Nutrition in clinical practice - the refeeding syndrome: illustrative cases and guidelines for prevention and treatment," 2008, Eur J Clin Nutr., 62(6): 687-94

### Summary of Invention

### Technical Problem

An object of the present invention is to provide a composition for use in preventing or suppressing refeeding syndrome (which is also referred to below as "the composition of the present invention").

### Solution to Problem

In order to achieve the above object, the present inventors conducted extensive research and found that the administration of a substance having an mTOR inhibitory action can inhibit refeeding syndrome via mTOR inhibitory action. The present inventors conducted further research based on this finding and have accomplished the present invention. The present invention includes the following.

Item 1.
   A composition for use in preventing or suppressing refeeding syndrome, the composition comprising an mTOR inhibitor.
Item 2.
   The composition according to Item 1, which is for use in preventing or suppressing hypophosphatemia.
Item 3.
   The composition according to Item 1 or 2, which is for use so that the composition is ingested or administered to a subject in an undernourished state.
Item 4.
   The composition according to any one of Items 1 to 3, which is for use so that the composition is ingested or administered at the time of refeeding.
Item 5.
   The composition according to any one of Items 1 to 4, wherein the mTOR inhibitor comprises rapamycin.
Item 6.
   A method for preventing or suppressing refeeding syndrome in a subject, the method comprising administering an mTOR inhibitor to the subject.

### Advantageous Effects of Invention

According to the present invention, refeeding syndrome can be prevented or suppressed.

### Brief Description of Drawings

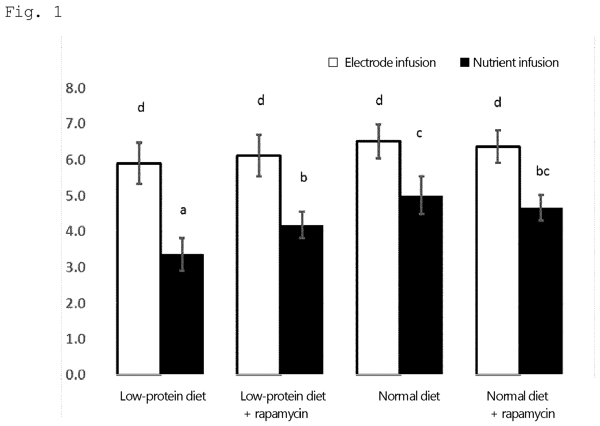

Fig. 1 is a graph showing the plasma phosphorus concentration (mg/dL) after administering a nutrient infusion preparation or an electrolyte infusion preparation at the time of refeeding. The results are shown as the mean ± standard deviation. n = 8. Different alphabetic letters indicate that there is a significant difference therebetween (p <0.05, Holm's multiple comparison).

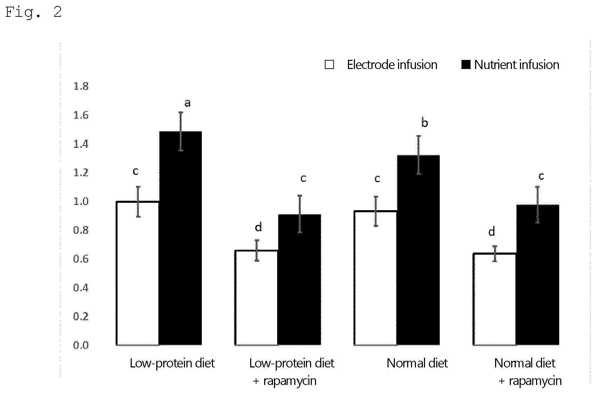

Fig. 2 is a graph showing the expression of phosphorylated mTOR protein in the liver. The results are shown as the mean ± standard deviation (n = 8). Different alphabetic letters indicate that there is a significant difference therebetween. An increase in expression of phosphorylated mTOR was observed in the rats fed with a low-protein diet when a nutritional composition is administered as refeeding. Different alphabetic letters indicate that there is a significant difference therebetween (p <0.05, Holm's multiple comparison).

### Description of Embodiments

The composition of the present invention comprises an mTOR inhibitor as an active ingredient.

The mTOR inhibitor can be selected from a wide range of known mTOR inhibitors. Examples of usable mTOR inhibitors include rapamycin (also referred to as "sirolimus") and rapalogs (also referred to as "rapamycin derivatives"), ATP-competitive mTOR kinase inhibitors, and foods containing a ketone body. The composition of the present invention preferably contains rapamycin. Examples of rapalogs include deforolimus, everolimus, and temsirolimus. Examples of ATP-competitive mTOR kinase inhibitors include Torin-1, Torin-2, and vistusertib. Examples of foods containing a ketone body include acetoacetic acid, 3-hydroxyacetic acid, salts thereof, and derivatives thereof. The composition of the present invention may contain only one mTOR inhibitor, or two or more mTOR inhibitors.

The mechanism of the mTOR inhibition includes, for example, a mechanism of suppressing the expression of mTOR mRNA and/or protein and a mechanism of suppressing mTOR activation. Examples of the suppression of mTOR activation include suppression of mTOR phosphorylation.

The subject to which the composition of the present invention is applied is not particularly limited. The composition of the present invention can be applied to humans or non-human animals. The composition of the present invention is safe for application to humans.

"Refeeding syndrome" is a general term for a series of metabolic complications that develop as a result of providing aggressive nutritional support for a subject in an undernourished state. Specific examples of symptoms of refeeding syndrome include hypophosphatemia, hypokalemia, hypomagnesemia, and vitamin B₁ deficiency. Ingestion or administration of the composition of the present invention can particularly prevent or suppress hypophosphatemia.

The composition of the present invention provides its pharmaceutical efficacy via mTOR inhibitory action. Accordingly, any substance that has mTOR inhibitory action can be used as an active ingredient of the composition of the present invention.

The composition of the present invention is preferably used so that the composition is ingested or administered to a subject in an undernourished state. The composition of the present invention is more preferably used so that the composition is ingested or administered at the time of refeeding. Using the composition of the present invention in such a manner can effectively prevent or suppress refeeding syndrome.

When the composition of the present invention is used so that the composition is ingested or administered at the time of refeeding, the composition of the present invention can be ingested or administered in combination with refeeding with a nutrient infusion preparation. In this case, the composition of the present invention can be ingested or administered before, simultaneously with, or after the refeeding.

When the composition of the present invention is ingested or administered before refeeding, the composition of the present invention can also be used as a prophylactic agent.

The composition of the present invention is preferably used for subjects in an undernourished state that have the possibility of developing refeeding syndrome.

Such subjects have, for example, at least one of the following (a) to (d), which are specified in the NICE guidelines:
(a) BMI less than 16 kg/m²;
(b) unintentional weight loss greater than 15% within the last 3-6 months;
(c) little or no nutritional intake for more than 10 days; and
(d) low levels of potassium, phosphate, or magnesium before refeeding, or
subjects have two or more of the following (e) to (h):
(e) BMI less than 18.5 kg/m²;
(f) unintentional weight loss greater than 10% within the last 3-6 months;
(g) little or no nutritional intake for more than 5 days; and
(h) a history of alcohol abuse or drugs, including insulin, chemotherapy, antacids, or diuretics.

Undernourished subjects can be classified into:
the marasmus type, in which protein and calorific value are both deficient; the kwashiorkor type, in which protein deficiency is more severe than calorific value deficiency; and the marasmus-kwashiorkor type, which is an intermediate type between the two.
The refeeding syndrome symptom suppressor of the present invention can effectively prevent or suppress refeeding syndrome by administration to marasmus-kwashiorkor-type undernourished subjects and kwashiorkor-type undernourished subjects.

The composition of the present invention can be a food or medicament.

The dosage form of the composition of the present invention is not particularly limited. For example, the composition can be in the form of a solid preparation, a semi-solid preparation, or a liquid preparation.

Examples of solid preparations include dusts, powders, and granules (e.g., granules and fine granules), balls, pills, tablets (e.g., sublingual tablets, orally disintegrating tablets, pastilles, and chewable tablets), capsules (e.g., hard capsules, soft capsules, and microcapsules), dry syrups, suppositories, film formulations, and sheet formulations.

The capsules can be liquid-filled capsules or capsules containing a solid agent, such as granules. The composition of the present invention may also be a lyophilized formulation.

Examples of semi-solid preparations include gel, jelly, gummy, cake-like, and paste preparations.

Examples of liquid preparations include solutions, suspensions, emulsions, syrups, elixirs, and injections.

The composition of the present invention may also be a spray of the powder and/or liquid preparation described above, or an aerosol thereof.

Further, the composition of the present invention may be a preparation with a controlled release rate of the active ingredient (a sustained-release formulation or a rapid-release formulation) .

As long as the composition of the present invention contains an mTOR inhibitor, there is no particular limitation. The mTOR inhibitor can be used alone or in combination with, for example, a pharmacologically or physiologically acceptable additive.

Examples of additives include stabilizers, preservatives, buffers, taste-masking agents, suspending agents, and emulsifiers. These additives can be appropriately added according to, for example, the dosage form. Such various additives can be used alone or in a combination of two or more.

When the composition of the present invention is a solid preparation, excipients, binders, disintegrants, lubricants, coating agents, and lipids can be used as carriers. Such various carriers can be used alone or in a combination of two or more.

Examples of the excipients include sugars or sugar alcohols, such as lactose, white sugar, glucose, sucrose, mannitol, sorbitol, and xylitol; starches, such as corn starch and potato starch; polysaccharides, such as crystalline cellulose (including microcrystalline cellulose); and silicon oxide or silicates, such as light anhydrous silicic acid and synthetic aluminium silicate.

Examples of the binders include soluble starches, such alpha starch and partial alpha starch; polysaccharides, such as agar, gum arabic, dextrin, sodium alginate, tragacanth gum, xanthan gum, hyaluronic acid, pectin, and sodium chondroitin sulphate; synthetic polymers, such as polyvinylpyrrolidone, polyvinyl alcohol, carboxyvinyl polymers, polyacrylic acid polymers, polylactic acid, and polyethylene glycol; and cellulose ethers, such as methyl cellulose, ethyl cellulose, carboxymethyl cellulose, sodium carboxymethylcellulose, hydroxyethylcellulose, hydroxypropylcellulose, and hydroxypropylmethylcellulose.

Examples of the disintegrants include calcium carbonate, carboxymethyl cellulose or salts thereof (e.g., carmellose, sodium carmellose, and calcium carmellose), polyvinylpyrrolidones (e.g., polyvinylpyrrolidone and crosslinked polyvinylpyrrolidone (crospovidone)), and low-substituted hydroxypropyl cellulose.

Examples of the lubricants include talc, magnesium stearate, and polyethylene glycol 6000.

Examples of the coating agents include sugars, cellulose derivatives, such as ethyl cellulose and hydroxymethyl cellulose, polyoxyethylene glycol, cellulose acetate phthalate, hydroxypropyl methyl cellulose phthalate, methyl methacrylate-(meth)acrylic acid copolymers, eudragit (a methacrylic acid-acrylic acid copolymer), and the like. The coating agent to be used can be an enteric soluble component, such as cellulose phthalate, hydroxypropyl methyl cellulose phthalate, and methyl methacrylate-(meth)acrylic acid copolymers, or can be a gastric-soluble component composed of a polymer containing a basic component, such as dialkylaminoalkyl (meth)acrylate, (e.g., eudragit).

Examples of the lipids include waxes (e.g., beeswax, carnauba wax, cocoa fat, lanolin, paraffin, and vaseline), long-chain fatty acid esters (e.g., saturated or unsaturated fatty acid alkyl esters and esters of fatty acids and polyhydric alcohols (e.g., poly C₂₋₄ alkylene glycols, glycerine, or polyglycerine) (e.g., glycerides)), hardened oils, higher alcohols (e.g., saturated aliphatic alcohols, such as stearyl alcohol, and unsaturated aliphatic alcohols, such as oleyl alcohol), higher fatty acids (e.g., linoleic acid, linoleic acid, oleic acid, and stearic acid), metal soaps (e.g., fatty acid metal salts such as sodium coconut oil fatty acid and calcium stearate).

When the composition of the present invention is a liquid preparation, oily carriers or aqueous carriers can be used. Examples of oily carriers include animal and vegetable oils (e.g., vegetable-based oils, such as jojoba oil, olive oil, palm oil, and cottonseed oil; and animal-based oils, such as squalane) and mineral oils (e.g., liquid paraffin and silicone oil). Examples of aqueous carriers include water (e.g., purified or sterile water, and distilled water for injection), physiological saline, Ringer's solution, glucose solution, water-soluble organic solvents (e.g., lower aliphatic alcohols, such as ethanol and isopropanol; (poly)alkylene glycols (e.g., ethylene glycol and polyethylene glycol); and glycerol), dimethylisosorbide, and dimethylacetamide.

When the composition of the present invention is a semi-solid preparation, the carrier to be used can be selected from the carriers for solid preparations and/or carriers for liquid preparations described above. Further, the semi-solid preparation may contain a lipid of such a solid formulation.

When the composition of the present invention is a medicament and administered to a standard adult (body weight: 65 kg), the composition is administered so that the daily dose of the active ingredient is preferably 0.1 to 20 mg, more preferably 1 to 8 mg, and even more preferably 2 to 4 mg. When the composition of the present invention is a food containing a ketone body, the dose can be set so that the ketone body dose accounts for about 5 to 20%, preferably 10 to 20%, and more preferably 15 to 20%, of the total energy consumption (which is estimated, for example, as an average of 2500 kcal from the "Dietary Reference Intakes for Japanese 2020" published by the Ministry of Health, Labour and Welfare.)

The composition of the present invention can be ingested or administered once or several times (e.g., 2 to 5 times) per day. The route of administration and the optimum dose are determined by considering the subject's condition (general condition, medical condition, etc.), age, sex, body weight, etc.

When the composition of the present invention is a medicament, the method for administration can be, for example, oral administration, drip infusion or injection (e.g., intravenous, intramuscular, or subcutaneous injection), eye drops, or intranasal or transdermal administration. When the composition of the present invention is administered by drip infusion, the composition may be mixed into a nutrient infusion preparation and administered.

When the composition of the present invention is a food, the method for administration to a subject includes, for example, oral administration and enteral administration (e.g., gastric fistula and intestinal fistula).

### Examples

The present invention is described below with reference to Examples. However, the present invention is not limited to these Examples, etc.

### Effect of rapamycin on blood phosphorus concentration after refeeding to protein-undernourished rats

Using male Wistar rats (7 weeks old at time of purchase; Charles River Laboratories Japan, Inc.), a group of rats fed with a purified diet having a protein content of 2.5% (referred to below as "low-protein diet") for at least 3 weeks, and a group of rats fed with a purified diet having a protein content of 20% (referred to below as "normal diet") for at least 3 weeks were prepared. Table 1 shows the formulation of each diet. Each animal had access to tap water ad libitum for at least 3 weeks. After fasting with ad libitum access to water for 24 hours, jugular vein catheterization was performed under isoflurane anesthesia, and the other end of the catheter was passed under the skin, exited from the back, and connected to a syringe containing physiological saline via a harness. The rats were then kept in individual metabolic cages with the catheter being open. Post-operatively, an antibiotic (ampicillin) was injected intramuscularly at a dose of 5 mg/100 µL/rat. After recovery from anesthesia, blood (400 µL) was collected from the catheter placed in the jugular vein, then injected into a tube containing sodium heparin, and stored on ice. The blood was promptly fractionated to obtain plasma, and the inorganic phosphorus concentration in the plasma was promptly measured using a Fuji Dry Chem Slide (produced by Fuji Film Corporation). The rats were grouped into predetermined groups using the obtained measurements and body weight data as indices.

The physiological saline was replaced with a nutrient infusion preparation or an electrolyte preparation at 5 p.m. to start administration to the animals. The nutrient infusion preparation used was Elneopa-NF No. 2 infusion (produced by Otsuka Pharmaceutical Factory Co., Ltd.). The electrolyte formulation used was an electrolyte infusion preparation composed of the electrolyte contained in Elneopa-NF No. 2 infusion. Table 2 shows the formulation of Elneopa-NF No. 2 infusion preparation. Immediately before administering these preparations, either a solvent (dimethyl sulfoxide, produced by Fujifilm Wako Pure Chemicals Corporation; referred to below as "DMSO") or a solution of 2 mg/mL of rapamycin (produced by Tokyo Chemical Industry Co., Ltd.) whose side effect of inhibiting muscle synthesis was known was intraperitoneally administered by bolus at a dose of 0.75 mL/kg in each case. For refeeding, the nutrient infusion preparation was continuously infused into the nutrient infusion preparation administration animals over a period of 16 hours so that nutrient infusion preparation was administered at a calorific value of 150 kcal (a liquid volume of 183 ml) per kilogram of body weight, whereas the electrolyte preparation was continuously infused into the electrolyte preparation administration animals over a period of 16 hours so that the electrolyte preparation was administered in a liquid volume of 183 mL per kilogram of body weight.

Starting at 9 a.m. on the day after the start of administration, blood was collected from the abdominal aorta by using a syringe under isoflurane anesthesia. The obtained blood was injected into vacuum blood collection tubes containing sodium heparin and stored on ice and then centrifuged (3000 rpm, 4°C, 15 min) to obtain plasma by fractionation. The concentration of inorganic phosphorus in the plasma was determined by an enzymatic method (Determiner L, produced by Hitachi Chemical Diagnostics Systems Co., Ltd.).

**Table 1**

| Starting materials | Unit | Normal diet | Low-protein diet |
|---|---|---|---|
| Casein | % (w/w) | 20 | 2.5 |
| L-methionine | | 0.32 | 0.04 |
| Beta corn starch | | 33.45 | 34.15 |
| Alpha corn starch | | 10 | 22 |
| Sucrose | | 21.73 | 26.81 |
| Corn oil | | 5 | 5 |
| Cellulose powder | | 5 | 5 |
| AIN-93G mineral mix | | 3.5 | 3.5 |
| AIN-93 vitamin mix | | 1 | 1 |
| Total | | 100 | 100 |

**Table 2**

| Nutrient infusion (1000 mL) | | |
|---|---|---|
| Component | | Content |
| Carbohydrate | Glucose | 175 g |
| | Sugar concentration | 17.5% |
| Electrolyte | Na⁺ | 50 mEq |
| | K⁺ | 27 mEq |
| | Mg²⁻ | 5 mEq |
| | Ca² | 5 mEq |
| | Cl | 50 mEq |
| | SO₄²⁻ | 5 mEq |
| | Acetate | 48 mEq |
| | L-lactate | 14 mEq |
| | Citrate³ | 12 mEq |
| | P | 6 mmol (187 mg) |
| Vitamin | Thiamine chloride hydrochloride | 3.84 mg |
| | Sodium riboflavin phosphate | 2.3 mg |
| | Pyridoxine hydrochloride | 3.675 mg |
| | Cyanocobalamin | 2.5 µg |
| | Nicotinic acid amide | 20 mg |
| | Panthenol | 7 mg |
| | Folic acid | 0.3 mg |
| | Biotin | 30 µg |
| | Ascorbic acid | 100 mg |
| | Vitamin A oil | 1650 Vitamin A unit |
| | Cholecalciferol | 2.5 ug |
| | Tocopherol acetate | 5 mg |
| | Phytonadione | 0.075 mg |
| Trace element | Iron (Fe) | 10 µmol |
| | Manganese (Mn) | 0.5 µmol |
| | Zinc (Zn) | 30 µmol |
| | Copper (Cu) | 2.5 µmol |
| | Iodine (I) | 0.5 µmol |
| Amino acid | Total free amino acid content: 20 g, 30 g, 40 g | 30 g |
| | Total nitrogen content: 3.13 g, 4.70 g, 6.27 g | 4.70 g |
| | Essential amino acid/ non-essential amino acid | 1.44 |
| | Branched chain amino acid content | 30 w/w% |
| Total calorie | | 820 kcal |
| Non-protein calorie | | 700 kcal |
| Non-protein calorie/nitrogen ratio | | 149 |

Fig. 1 and Table 3 show the results. "NPD (refeeding)" indicates the plasma phosphorus concentration in the rats fed with the normal diet after refeeding with the nutrient infusion. "NPD (refeeding + rapamycin)" indicates the plasma phosphorus concentration in the rats fed with the normal diet after administration of rapamycin and refeeding with the nutrient infusion. "LPD (refeeding)" indicates the plasma phosphorus concentration in the rats fed with the low-protein diet after refeeding with the nutrient infusion. "LPD (refeeding + rapamycin)" indicates the plasma phosphorus concentration in the rats fed with the low-protein diet after administration of rapamycin and refeeding with the nutrient infusion.

"NPD (electrolyte)" indicates the plasma phosphorus concentration in the rats fed with the normal diet after administration of the electrolyte infusion. "NPD (electrolyte + rapamycin)" indicates the plasma phosphorus concentration in the rats fed with the normal diet after administration of rapamycin and administration of the electrolyte infusion. "LPD (electrolyte)" indicates the plasma phosphorus concentration in the rats fed with the low-protein diet after administration of the electrolyte infusion. "LPD (electrolyte + rapamycin)" indicates the plasma phosphorus concentration in the rats fed with the low-protein diet after administration of rapamycin and administration of the electrolyte infusion. In the present specification, the plasma inorganic phosphorus concentrations are expressed as the mean ± standard deviation.

In all groups of combinations (n = 8, Holm's multiple comparisons), there was no significant difference in plasma inorganic phosphorus concentration before refeeding.

There was no significant difference in blood phosphorus concentration among the rats fed with the electrolyte infusion after administration of the electrolyte infusion ("NPD (electrolyte)" versus "LPD (electrolyte)"). Although refeeding of the rats fed with the normal diet with the nutrient infusion preparation also slightly reduced the plasma phosphorus concentration ("NPD (electrolyte)" versus "NPD (refeeding)"), refeeding of the rats fed with the low-protein diet with the nutrient infusion preparation more significantly affected the reduction of the plasma phosphorus concentration ("LPD (refeeding)" versus "NPD (refeeding)").

Thus, the symptoms of refeeding syndrome were reproduced. That is, solely resulting in a low nutrition state due to ingestion of a low-protein diet does not develop hypophosphatemia, and additional refeeding with a nutrient infusion develops hypophosphatemia. On the other hand, the phosphorus concentration in rats to which rapamycin and the electrolyte infusion were administered was not so different from the concentration in rats to which the electrolyte infusion was administered without administering rapamycin, regardless of which diet was given. When rapamycin was administered and the rats were refed with the nutrient infusion, the rats fed with the normal diet showed an equivalent level of blood phosphorus concentration as the rats to which no rapamycin was administered, whereas the rats fed with the low-protein diet showed a significantly high level of blood phosphorus concentration due to the administration of rapamycin ("LPD (refeeding)" versus "LPD (refeeding + rapamycin)").

The above results show that rapamycin affects the blood phosphorus concentration when the subjects are refed with a nutrient infusion, not an electrolyte infusion, and that the reduction of blood phosphorus concentration is suppressed only when the rats fed with a low-protein diet are refed.

**Table 3**

| | Test diet | Administration of rapamycin | Preparation administered at the time of refeeding | Plasma phosphorus concentration after administration (mg/dL) |
|---|---|---|---|---|
| LPD (electrolyte) | Low-protein diet | No | Electrolyte infusion | 5.9±0.6 |
| LPD (refeeding) | | No | Nutrient infusion | 3.4±0.5 |
| LPD (electrolyte + rapamycin) | | Yes | Electrolyte infusion | 6.110.6 |
| LPD (refeeding + rapamycin) | | Yes | Nutrient infusion | 4.2±0.4 |
| NPD (electrolyte) | Normal diet | No | Electrolyte infusion | 6.5±0.5 |
| NPD (refeeding) | | No | Nutrient infusion | 5.0±0.5 |
| NPD (electrolyte + rapamycin) | | Yes | Electrolyte infusion | 6.4±0.4 |
| NPD (refeeding + rapamycin) | | Yes | Nutrient infusion | 4.7±0.4 |

| | | | | |
|---|---|---|---|---|
| Mean ± standard deviation, n = 8 | | | | |

### Effect of rapamycin on phosphorylated mTOR protein expression in undernourished rats

Using protein-undernourished rats and normal rats, liver tissues after intraperitoneal administration of rapamycin or a solvent (DMSO) and refeeding were obtained. The expression of phosphorylated mTOR protein in the liver of the animals in each group was determined using a PathScan (registered trademark) Phospho-mTOR (Ser2448) Sandwich ELISA Kit (#7976, produced by Cell Signaling Technology). The measurement data obtained were corrected using the liver protein concentrations. Test samples were obtained in the animal tests described in the Examples.

Fig. 2 shows the results. The value of each group is expressed in terms of relative ratio of each group with the expression of phosphorylated mTOR protein in the protein-undernourished rats fed with the solvent and then the electrolyte infusion being defined as 1. When a solvent was administered (i.e., rapamycin was not administered), the rats refed with the nutrient infusion showed an increase in expression of phosphorylated mTOR protein in the liver as compared to the rats to which the electrolyte infusion was administered. This increase was more prominent in the protein-undernourished rats. In contrast, when rapamycin was administered, the expression of phosphorylated mTOR protein was reduced in all the rapamycin administration groups; and when the rats were refed with the nutrient infusion, the expression of phosphorylated mTOR did not change depending on the nutritional state of the animals.

Under the conditions in which protein-undernourished rats were refed, a significant increase in expression of phosphorylated mTOR protein, which is an mTOR activator, and a significant decrease in blood phosphorus concentration were observed. Since the administration of rapamycin inhibited mTOR protein phosphorylation and also suppressed the reduction of blood phosphorus concentration, the relationship between the mTOR signaling pathway and the reduced blood phosphorus concentration at the time of refeeding was strongly suggested.

## Claims

1. A composition for use in preventing or suppressing refeeding syndrome, the composition comprising an mTOR inhibitor.

2. The composition according to claim 1, which is for use in preventing or suppressing hypophosphatemia.

3. The composition according to claim 1 or 2, which is for use so that the composition is ingested or administered to a subject in an undernourished state.

4. The composition according to any one of claims 1 to 3, which is for use so that the composition is ingested or administered at the time of refeeding.

5. The composition according to any one of claims 1 to 4, wherein the mTOR inhibitor comprises rapamycin.

6. A method for preventing or suppressing refeeding syndrome in a subject, the method comprising administering an mTOR inhibitor to the subject.
